# EUROPÄISCHE PATENTANMELDUNG

(11) **EP 0 656 538 A1**
(43) Veröffentlichungstag der Anmeldung: **07.06.1995**
(21) Anmeldenummer: 94117629.9
(22) Anmeldetag: 08.11.1994
(51) Int. Cl.: G01N 33/00, G01N 27/407

(54) **Gassensor**

(30) Priorität: 22.11.1993 DE 4339737
(71) Anmelder: SIEMENS AKTIENGESELLSCHAFT, D-80333 München (DE)
(72) Erfinder: Gerblinger, Josef, Dr., D-86637 Wertingen (DE); Lampe, Uwe, Dr., D-21614 Buxtehude (DE); Meixner, Hans, Prof. Dr., D-85540 Haar (DE)

(57) **Zusammenfassung**

2.1. Aufgrund ihrer im Millisekundenbereich liegenden Ansprechgeschwindigkeit eignen sich Sauerstoffsensoren auf der Basis gesputterter Titanatschichten insbesondere als Lamda-Sonden zur zylinderselektiven Regelung der Luftzahl. Da chlorhaltige Kohlenwasserstoffe und Chlorgas die nur wenige µm dicken Titanatschichten in irreversibler Weise schädigen können, dürfen diese Abgaskomponenten unter keinen Umständen mit dem sauerstoffempfindlichen Element in Kontakt kommen.

2.2. In einem erfindungsgemäßen Sensor deckt eine poröse Schutzschicht (34) das gasempfindliche Element (33) ab. Die mit Hilfe eines Siebdruckverfahrens hergestellte Schutzschicht (34) besteht aus einem Material, das ein das Sensorelement (33) schädigendes Gas chemisch bindet. Eine aus Titandioxid, Bariumtitanat oder Kalziumtitanat bestehtende Schutzschicht (34) kann beispielsweise die Wechselwirkung von chlorhaltigen Kohlenwasserstoffen und Chlorgas mit dem in schnellen Sauerstoffsensoren verwendeten Strontiumtitanat verhindern.

3. Lamda-Sonde

## Beschreibung

Die im Bereich des Umweltschutzes verwendeten Gassensoren sind in der Regel einem Gemisch von Gasen mit stark unterschiedlicher Reaktivität ausgesetzt. Besteht das gasempfindliche Element aus einem Metalloxid, so findet in den meisten Fällen ab einer bestimmten Temperatur eine Wechselwirkung zwischen einer Komponente des Gasgemischs und dem Sensormaterial statt. Bei dieser Wechselwirkung kann es sich um die gewünschte reversible Reaktion mit der nachzuweisenden Gaskomponente (z. B. Volumenreaktion, Adsorptions- bzw. Desorptionsprozesse) oder um eine chemische Reaktion handeln. Da vor allem chemische Reaktionen eine Zerstörung der oft nur wenige µm dicken Metalloxidschicht bewirken bzw. deren Eigenschaften in irreversibler Weise ändern können, müssen solche Reaktionen im Hinblick auf die geforderte Langzeitstabilität der Gassensoren unbedingt vermieden werden.

Im Abgas von Kraftfahrzeugen erweisen sich chlorhaltige Kohlenwasserstoffe (CₓH_{y}Cl_{z}) und Chlorgas (Cl₂) als besonders reaktiv. Ursache für deren Emission sind die im Benzin vorhandenen oder diesem beigemischten Chlorverbindungen. Probleme bereitet die Wechselwirkung von Chlor mit dem beispielsweise in schnellen Lamda-Sonden verwendeten Strontiumtitanat (SrTiO₃). Den letztendlich zur Zerstörung der Lamda-Sonde führenden Abbau der sauerstoffempfindlichen Schicht kann man hier im wesentlichen auf die Reaktion von Chlor mit dem Titan des Strontiumtitanats zu dem flüchtigen Titantetrachlorid (TiCl₄) zurückführen. Ein ähnliches Verhalten gegenüber Chlor zeigen auch andere Sensormaterialien wie beispielsweise Bariumtitanat (BaTiO₃) oder Titandioxid (TiO₂).

Die US-A- 4,347,732 beschriebt einen Gasdetektor, dessen Sensorelement von einer porösen Passivierungsschicht abgedeckt ist. Da die aus einem Edelmetall oder einem Zeolith bestehende Passivierungsschicht als molekulares Sieb wirkt, können nur Moleküle bestimmter Gase zum Sensorelement gelangen. So ist beispielsweise eine einen effektiven Porendurchmesser d ≦ 0,3 nm aufweisende Zeolith-3A-Schicht für Wasserstoff (H₂), Sauerstoff (O₂), Kohlenmonoxid (CO) und Ammoniak (NH₃), aber nicht für Schwefelwasserstoff (H₂S), Methan (CH₄), Schwefeldioxid (SO₂) oder Kohlendioxid (CO₂) durchlässig.

Gegenstand der Erfindung ist ein Gassensor, den man einer aggressive Gase enthaltenden Atmosphäre längere Zeit ohne Schaden zu nehmen aussetzen kann. Der Sensor soll insbesondere unempfindlich sein gegenüber den im Abgas von Verbrennungsmotoren und Feuerungsanlagen vorhandenen Chlor- und Schwefelverbindungen. Diese Aufgabe wird erfindungsgemäß durch einen Gassensor nach Patentanspruch 1 gelöst.

Der mit der Erfindung erzielbare Vorteil besteht insbesondere darin, daß sich die Lebensdauer dünner sauerstoffempfindlicher Strontium- und Bariumtitanatschichten deutlich erhöht. Dies ermöglicht den Bau von schnellen Sauerstoffsensoren, die man beispielsweise als Lamda-Sonde zur zylinderselektiven Regelung der Luftzahl einsetzen kann.

Die abhängigen Ansprüche betreffen vorteilhafte Weiterbildungen und Ausgestaltungen der im folgenden anhand der Zeichungen erläuterten Erfindung. Hierbei zeigt:
- Fig. 1: den schematischen Aufbau der Abgasanlage eines Verbrennungsmotors
- Fig. 2 und 3: Ausführungsbeispiele von Sauerstoffsensoren
- Fig. 4: eine schnelle Lamda-Sonde
- Fig. 5: einen erfindungsgemäßen Gassensor im Querschnitt
Die Abgasanlage eines modernen Kraftfahrzeugs soll die während des Motorbetriebs entstehenden Schadstoffe weitgehend abbauen und die verbleibenden Restgase an einer für den Gebrauch des Fahrzeugs günstigen Stelle möglichst geräuscharm in die Atmosphäre abgeben. Sie besteht üblicherweise aus einem motornah eingebauten Katalysator 1, einem oder mehreren Schalldämpfern 2 und einem Rohrsystem 3, das die einzelnen Komponenten mit den im Zylinderkopf des Motors 4 vorhandenen Abgasaustrittsöffnungen verbindet. Die vor dem Katalysator 1 im Auspuffrohr 3 verschraubte Lamda-Sonde 5 mißt den Sauerstoffpartialdruck im Abgas. Ihr Ausgangssignal wird an das nicht dargestellte Steuergerät der elektronischen Einspritzung weitergegeben, das die Kraftstoffzumessung in Abhängigkeit vom jeweils gemessenen Sauerstoffpartialdruck korrigiert.

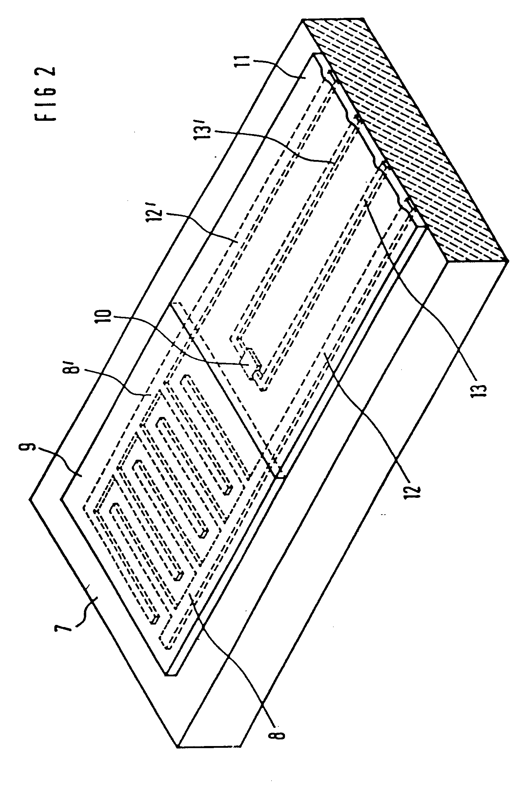

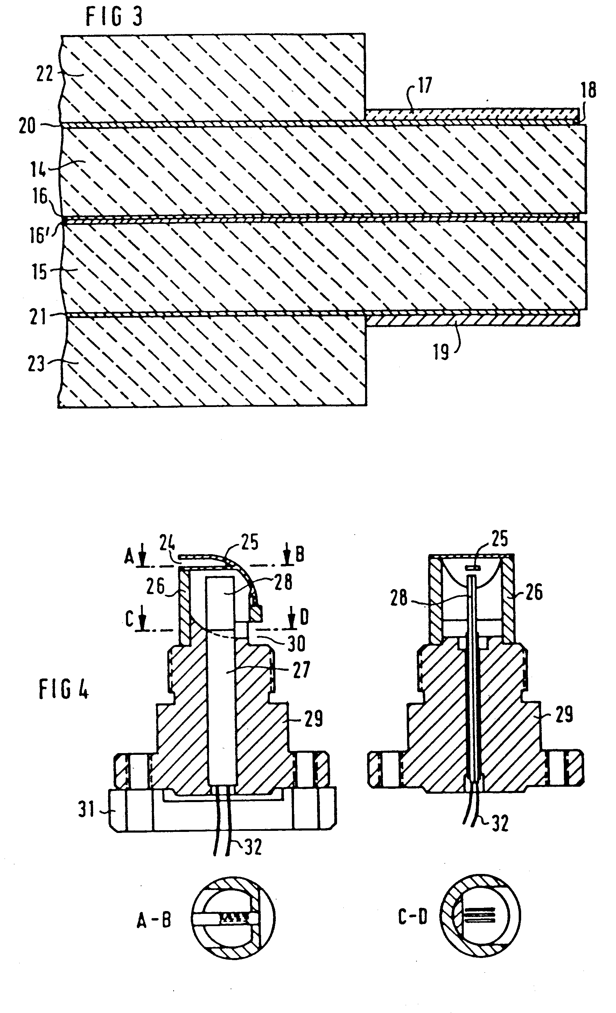

Konventionelle Sauerstoffsensoren auf der Basis des ionenleitenden Zirkondioxids eignen sich nicht zur zylinderselektiven Lamda-Regelung, da sie nur vergleichsweise langsam auf Änderungen des Sauerstoffpartialdrucks ansprechen. Die Lamda-Sonde 5 ist deshalb mit einem der in den Figuren 2 und 3 dargestellten Sensoren ausgestattet, deren Ansprechzeit nur wenige Millisekunden beträgt.

Das Substrat 7 des Sauerstoffsensors nach Figur 2 besteht beispielsweise aus Magnesium-, Silizium- oder Aluminiumoxid. Auf dem Substrat 7 sind zwei eine Interdigitalstruktur bildende Platinelektroden 8 und 8' eine diese Elektroden kurzschließende Strontium- oder Bariumtitanatschicht 9 sowie ein Temperaturfühler 10 angeordnet. Die Passivierungsschicht 11 aus Glas oder Siliziumoxid schirmt die den Elektroden 8 und 8' und dem Temperaturfühler 10 jeweils zugeordneten Anschlußleitungen 12 und 12' bzw. 13 und 13' von dem im Abgas vorhandenen Restsauerstoff ab. Als Heizelement findet eine auf der Rückseite des Substrats 7 angeordnete Widerstandsschicht aus Platin Verwendung (nicht dargestellt).

Bei dem in Figur 3 im Schnitt dargestellten Sauerstoffsensor dienen die zwischen den Al₂O₃-Substraten 14 und 15 angeordneten Platinmäander 16 und 16' als Heizelemente. Auf den den Schichten 16 und 16' jeweils gegenüberliegenden Flächen der Substrate 14 bzw. 15 sind die sauerstoffsensitive SrTiO₃- oder BaTiO₃-Schicht 17 und die Interdigitalelektroden 18 bzw. der ebenfalls aus Platin bestehende Temperatursensor 19 sowie deren Anschlußelektroden 20 bzw. 21 angeordnet. Die in Fig. 3 mit 22 und 23 bezeichneten Al₂O₃-Schichten sollen die katalytischen Wirkungen der den Interdigitalelektroden 18 und dem Temperatursensor 19 jeweils zugeordneten Anschlußleitungen 20 bzw. 21 unterdrücken.

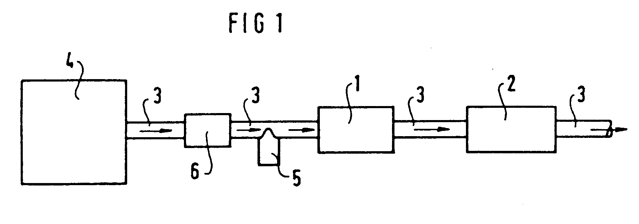

Das in Fig. 4 dargestellte Edelstahlgehäuse der in Fig. 1 mit 5 bezeichneten Lamda-Sonde besteht aus zwei Teilen, wobei der die Gaseintrittsöffnung 24 und einen Metallsteg 25 enthaltende Gehäusekopf 26 auf dem mit einer Bohrung 27 zur Aufnahme des Sauerstoffsensors 28 versehenen Grundkörper 29 befestigt ist. Vor dem Verschweißen der beiden Teile 26 und 29 wird der Sensor 28 in der Bohrung 27 des Grundkörpers 29 verklebt. Nach der Montage befindet sich der Sensorkopf in einem S-förmig gekrümmten Strömungskanal, der die Gaseintrittsöffnung 24 mit der Gasaustrittsöffnung 30 verbindet. Im linken Teil der Fig. 4 ist zusätzlich noch die die Bohrung 27 des unteren Gehäuseteils 29 abschließende Keramikplatte 31 gezeigt. Sie enthält mehrere Kanäle, durch die man die der Kontaktierung des Sauerstoffsensors 28 dienenden Anschlußdrähte 32 nach außen führt.

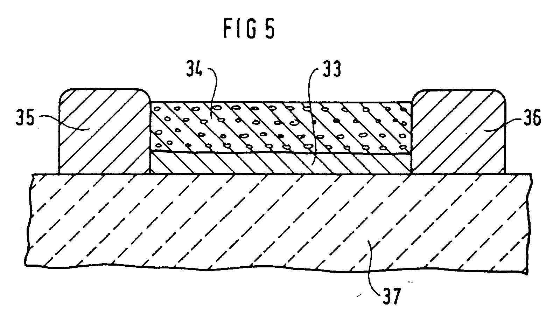

Um eine Wechselwirkung der nur etwa 1 bis 2 µm dicken Sputterschicht 33 aus Strontium- oder Bariumtitanat mit den im Abgas vorhandenen chlorhaltigen Kohlenwasserstoffen bzw. Chlorgas und Schwefeldioxid zu verhindern, ist der in Fig. 5 im Schnitt dargestellte Sensor mit einer den sauerstoffempfindlichen Bereich abdeckenden Schutzschicht 34 ausgestattet. Die mit Hilfe eines Siebdruckverfahrens hergestellte und damit poröse Schutzschicht 34 besteht aus einem Material, das hinsichtlich der Reaktion mit den die Sputterschicht 33 schädigenden Gasen annähernd die gleichen chemischen Eigenschaften besitzt wie das sauerstoffempfindliche Titanat. Im einfachsten Fall fertigt man das Sensorelement 33 und die Schutzschicht 34 aus demselben Material. Die Schutzschicht 34 eines SrTiO₃-Sensors besteht somit vorzugsweise ebenfalls aus Strontiumtitanat, so daß sich unter Einwirkung von chlorhaltigen Kohlenwasserstoffen oder Chlorgas das flüchtige Titantetrachlorid (TiCl₄) bildet. Als Schutzschichtmaterial eignen sich in diesem Fall aber auch andere titanhaltige Verbindungen wie Titandioxid (TiO₂), Bariumtitanat (BaTiO₃) oder Kalziumtitanat (CaTiO₃).

In Fig. 5 sind die die gassensitive Schicht 33 kontaktierenden Elektroden mit 35 bzw. 36 und das Sensorsubstrat mit 37 bezeichnet.

Der Widerstand der Schutzschicht 34 wird im wesentlichen durch deren Porosität bestimmt. Diese läßt sich im Falle einer siebgedruckten Dickschicht über den Anteil von Harzen in der Ausgangspaste variieren. Die obere Grenze für die Porosität ist hierbei durch die Forderung nach einer möglichst vollständigen Ausreaktion der den Sensor schädigenden Gase und der Forderung nach mechanischer Stabilität gegeben. Aus meßtechnischen Gründen sollte man außerdem darauf achten, daß der über die Porosität einstellbare Widerstand der Schutzschicht 34 um mindestens ein Faktor 100 größer ist als der Widerstand der gassensitiven Schicht 33.

## Patentansprüche

1. Gassensor mit einem gasempfindlichen Sensorelement (33), einem das Sensorelement (33) kontaktierenden Elektrodensystem (35, 36) und einer das Sensorelement (33) bedeckenden Schutzschicht (34), wobei zumindest die Schutzschicht (34) einem Gasgemisch ausgesetzt ist,
**dadurch gekennzeichnet,**
daß die Schutzschicht (34) aus einem Material besteht, das eine das Sensorelement (33) schädigende Komponente des Gasgemischs chemisch bindet.

2. Gassensor nach Anspruch 1,
**gekennzeichnet durch**
eine siebgedruckte Schutzschicht (34).

3. Gassensor nach Anspruch 1 oder 2,
**dadurch gekennzeichnet,**
daß die Schutzschicht (34) aus einem mit Chlorgas, chlorhaltigen Kohlenwasserstoffen oder Schwefeldioxid reagierenden Material besteht.

4. Gassensor nach Anspruch 3,
**dadurch gekennzeichnet,**
daß die Schutzschicht (34) aus einer titanhaltigen Verbindung besteht.

5. Gassensor nach Anspruch 4,
**dadurch gekennzeichnet,**
daß die Schutzschicht (34) aus Titandioxid, Bariumtitanat, Kalziumtitanat oder Strontiumtitanat besteht.

6. Gassensor nach einem der Ansprüche 1 bis 5,
**dadurch gekennzeichnet,**
daß das Sensorelement (33) aus einem halbleitenden Metalloxid besteht.

7. Gassensor nach Anspruch 6,
**dadurch gekennzeichnet,**
daß das Sensorelement (33) aus Bariumtitanat, Strontiumtitanat oder Galliumoxid besteht.

8. Gassensor nach einem der Ansprüche 1 bis 7,
**dadurch gekennzeichnet,**
daß die Schutzschicht (34) und das Sensorelement (33) aus dem gleichen Material bestehen und die Schutzschicht (34) als poröse Dickschicht ausgebildet ist.

9. Gassensor nach einem der Ansprüche 1 bis 8,
**dadurch gekennzeichnet,**
daß der elektrische Widerstand der Schutzschicht (34) um mindestens einen Faktor (100) größer ist als der elektrische Widerstand des Sensorelements (33).

10. Verwendung eines Gassensors nach einem oder mehreren der vorhergehenden Ansprüche zur Messung des Sauerstoffpartialdrucks im Abgas einer Brennkraftmaschine oder einer Verbrennungsanlage.
